# EUROPEAN PATENT APPLICATION

(11) **EP 3 123 973 A1**
(43) Date of publication of application: **01.02.2017**
(21) Application number: 16183287.8
(22) Date of filing: 28.08.2013
(51) Int. Cl.: A61B 18/14, A61B 17/00, A61M 25/00, A61M 25/01, A61B 18/00, A61M 25/02

(54) **RENAL RF ABLATION SYSTEM WITH A MOVABLE VIRTUAL ELECTRODE AND RELATED METHODS OF USE**

(30) Priority: 28.08.2012 US 201261694100 P
(62) Divisional of application: 13763134.7
(71) Applicant: Boston Scientific Scimed, Inc., Maple Grove, MN 55311-1566 (US)
(72) Inventor: Zhou, Pu, Trabuco Canyon, CA 92679 (US); Wang, Huisun, Maple Grove, MN 55311 (US); Cao, Hong, Maple Grove, MN 55311 (US)
(74) Representative: Vossius & Partner Patentanwälte Rechtsanwälte mbB

(57) **Abstract**

A renal nerve ablation system (200) may comprise a guide member (202) having a distal section (203) with a plurality of openings (208) formed therein, the distal section (203) being designed to shift between a first configuration and a second configuration and including one or more loops when in the second configuration. The system (200) may comprise an electrode catheter (210) slidably disposable within the guide member (202) and a mandrel removably disposable within the guide member (202), the mandrel being designed to hold the distal section (203) in the first configuration.

## Description

### Cross-Reference to Related Application

This application claims priority under 35 U.S.C. §119 to U.S. Provisional Application Serial No. 61/694,100, filed August 28, 2012, the entirety of which is incorporated herein by reference.

### Technical Field

This disclosure relates to devices and methods for intravascular neuromodulation. More particularly, the technologies disclosed herein relate to apparatus, systems, and methods, for achieving tissue modulation such as renal nerve ablation.

### Background

Certain treatments may require the temporary or permanent interruption or modification of select nerve function. One example treatment is renal nerve ablation, which is sometimes used to treat conditions related to congestive heart failure or hypertension. The kidneys produce a sympathetic response to congestive heart failure, which, among other effects, increases the undesired retention of water and/or sodium. Ablating some of the nerves running to the kidneys may reduce or eliminate this sympathetic function, which may provide a corresponding reduction in the associated undesired symptoms.

Many nerves, including renal nerves, run along the walls of or in close proximity to blood vessels and thus can be accessed intravascularly through the walls of the blood vessels. In some instances, it may be desirable to ablate perivascular nerves and/or tissue using radiofrequency (RF), ultrasonic, laser or microwave energy.

Some conventional ablation devices for hypertension include an ablation catheter having single electrode at the distal tip, used to deliver RF energy, for example, to the target issue. In some aspects, it may be difficult to obtain a controlled lesion pattern because the ablation catheter may not be easily controlled so that it can be pointed at the desired locations consistently and accurately. Recent advancements, however, provide a solution including a multi-electrode based ablation device. With the difficulty of reliable positioning and even distribution of RF energy to each electrode, providing even ablation results at different locations is a difficult task.

Therefore, there remains a need for improvement and/or alternatives in providing systems and methods for renal tissue ablation.

### Summary

The disclosure is directed to several alternative designs, materials, and methods of manufacturing medical device structures and assemblies.

Accordingly, some embodiments pertain to a tissue ablation device, including an elongate guide member having a distal section defined by a sidewall defining a lumen and having a plurality of openings such that the distal section has a helical shape in a released configuration. In addition, the device may include an elongate member having a distal end and slideable within the distal section of the guide member and having an ablation element disposed thereon. In one embodiment, the elongate guide member may bias to the released configuration. In addition, tissue ablation device may include a straightening member having a distal section that, when functionally engaged with the distal section of the guide member, moves the distal section from the released configuration to a restrained configuration where the distal section is substantially straight. In an exemplary embodiment, the straightening member may include a guide catheter having a lumen sized for the insertion of the guide member. In some other embodiments, the straightening member may have a size to be inserted within the guide member. Such examples may include a mandrel as the straightening member. Further, the helical section in the released configuration may include at least one loop. Still further, the helical section may also include at least two loops in the released configuration. Next, the plurality of openings may include a plurality of apertures that, when the distal section is in the released configuration, face radially out from the device. The plurality of apertures may be spaced from each other at a uniform distance. In one embodiment, the plurality of apertures may include at least three apertures; however, some other embodiments may include at least six apertures.

Some other embodiments pertain to a tissue ablation device having a plurality of pores such that the pores may be distributed along at least 80% of the length of the distal section of the guide member. In addition, the pores may be distributed circumferentially and longitudinally along the distal section of the guide member. Each of the pores has a maximum dimension of 0.05 inch. Further, the elongate member of the tissue ablation device, as discussed above, may include a shaft such that the shaft has a diameter less than the diameter of the ablation element. In one embodiment, the elongate member may further include a second ablation element disposed longitudinally from the ablation element. The ablation element may include an RF ablation element, an ultrasound ablation element, a cryogenic ablation element, a microwave ablation element, or the like.

Some instances also pertain to a method of using the tissue ablation device. The method includes moving the elongate guide member to a body lumen such that the elongate guide member may be positioned to the released configuration. Further, the elongate member may be advanced distally until the ablation element is at a first position in the distal section of the elongate guide member proximate one of the plurality of openings. Still further, the ablation element may be activated at the first position The method may further include moving the elongate member until the ablation element is at a second position different from the first position in the distal section of the elongate guide member proximate one of the plurality of openings, and activating the ablation element at the second position. In some embodiments, the method may also include moving the elongate member until the ablation element is at a third position different from the first position and the second position in the distal section of the elongate guide member proximate one of the plurality of openings; and activating the ablation element at the third position. Here, the second position is equidistant from the first position and the third position. In addition, the method may include providing saline through the lumen of the elongate guide member.

The following exemplary embodiments are disclosed:
Embodiment 1: A renal nerve ablation system, comprising:
   an elongate guide member having a distal section with a plurality of openings formed therein;
   wherein the distal section is designed to shift between a helical configuration and a restrained configuration; and
   an electrode catheter slidably disposed within the elongate guide member, the electrode catheter having an ablation element disposed thereon.
Embodiment 2: The renal nerve ablation system of embodiment 1, wherein the elongate guide member is biased to the helical configuration.
Embodiment 3: The renal nerve ablation system of any one of embodiments 1-2, further comprising a straightening member having a distal portion that, when coaxially engaged with the distal section of the guide member, moves the distal section from the helical configuration to the restrained configuration.
Embodiment 4: The renal nerve ablation system of embodiment 3, wherein the straightening member includes a guide catheter having a lumen sized for insertion of the guide member therein.
Embodiment 5: The renal nerve ablation system of embodiment 3, wherein the straightening member includes a mandrel slidably disposed within the elongate guide member.
Embodiment 6: The renal nerve ablation system of any one of embodiments 1-5, wherein the distal section of the guide member comprises at least one loop when in the helical configuration.
Embodiment 7: The renal nerve ablation system of any one of embodiments 1-6, wherein the distal section of the guide member comprises at least two loops when in the helical configuration.
Embodiment 8: The renal nerve ablation system of any one of embodiments 1-7, wherein the plurality of openings face radially outward when the guide member is in the helical configuration.
Embodiment 9: The renal nerve ablation system of any one of embodiments 1-8, wherein the plurality of openings are spaced from each other at a uniform distance.
Embodiment 10: The renal nerve ablation system of any one of embodiments 1-9, wherein the plurality of openings include a plurality of apertures.
Embodiment 11: The renal nerve ablation system of any one of embodiments 1 -9, wherein the plurality of openings include a plurality of pores.
Embodiment 12: The renal nerve ablation system of any one of embodiments 1-11, wherein the electrode catheter further comprises a second ablation element disposed longitudinally from the ablation element.
Embodiment 13: The renal nerve ablation system of any one of embodiments 1-12, wherein the ablation element includes an RF ablation element.
Embodiment 14: A renal nerve ablation system, comprising:
   a guide member having a distal section with a plurality of openings formed therein;
   wherein the distal section is designed to shift between a first configuration and a second configuration;
   wherein the distal section is biased to be in the second configuration;
   wherein the distal section includes one or more loops when in the second configuration;
   an electrode catheter slidably disposed within the guide member, the electrode catheter having an ablation element disposed thereon; and
   a mandrel removably disposed within the guide member, the mandrel being designed to hold the distal section in the first configuration.
Embodiment 15: The system of embodiment 14, wherein the second configuration is a helical configuration.

The summary of some example embodiments in not intended to describe each disclosed embodiment or every implementation of the disclosure.

### Brief Description of the Drawings

The present disclosure may be more completely understood in consideration of the following detailed description of various embodiments in connection with the accompanying drawings, in which:
FIG. 1 is a schematic view illustrating an exemplary tissue ablation system, according to embodiments of the present disclosure.
FIGS. 2A and 2B illustrate a portion of a tissue ablation device, in accordance with an embodiment of the present disclosure.
FIGS. 3A and 3B illustrate another embodiment of the tissue ablation device, according to the present disclosure.
FIGS. 4A and 4B illustrate yet another embodiment of the tissue ablation device, according to the present disclosure.
FIGS. 5A and 5B illustrate an exemplary method of using the tissue ablation device, according to an embodiment of the present disclosure.
FIG. 6 illustrates a portion of a tissue ablation device, in accordance with an embodiment of the present disclosure.

While embodiments of the present disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the present disclosure.

### Detailed Description

For the following defined terms, these definitions shall be applied, unless a different definition is given in the claims or elsewhere in the specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimension ranges and/or values pertaining to various components, features, and/or specifications are disclosed, one of skill in the art, incited by the present disclosure, would understand desired dimensions, ranges and/or values many deviate from those expressly disclosed.

As used in this specification and the appended claims, the singular forms "a," "an," and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary.

Embodiments of the present disclosure describe an ablation device adapted to ablate renal artery tissue. In general, the ablation device may include a distal section having a pre-defined released configuration. In addition, the distal section may be altered to a restrained configuration for delivery purposes. The ablation device in released configuration may be configured to conform to the target body lumen. Further, the ablation device may include an ablation element, which when adapted with the released configuration of ablation device may provide a pre-defined lesion pattern. As used herein, a "lesion" may be a change in tissue structure or function due to injury (e.g. tissue damage caused by the ultrasound or RF heating. The lesion is due to heating by energy such as ultrasound or RF energy). Also, it will be understood that "proximal" and "distal", as used in this disclosure, refers to positions or directions nearer to or farther from the user, respectively.

While the devices and methods described herein are discussed relative to renal tissue ablation treating hypertension, it is contemplated that the devices and methods may be used in other applications and/or body locations where tissue ablation is desired. For example, the devices and methods disclosed herein may be used for sympathetic nerve modulation.

In some instances, it may be desirable to employ renal artery for deployment of the ablation device to ablate renal tissue. It may be, however, understood that other target sites may also be employed to deploy the ablation device.

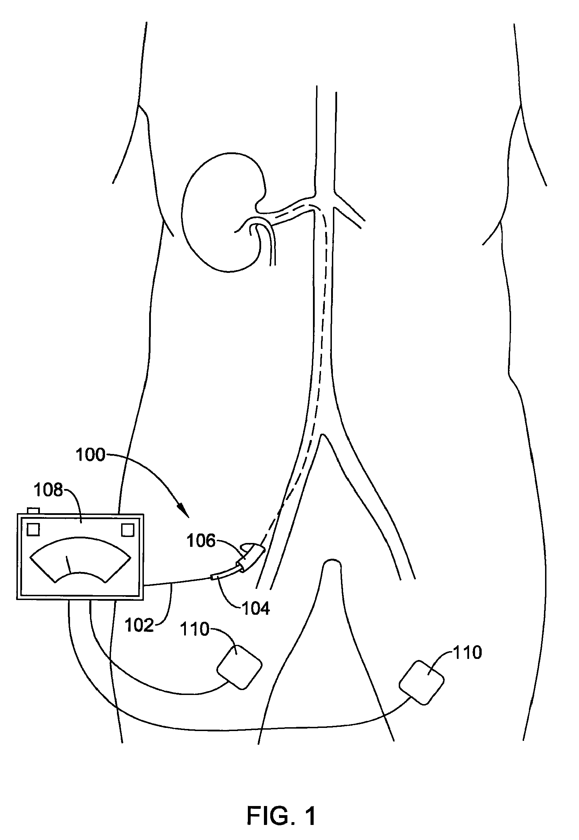

FIG. 1 is a schematic view illustrating an exemplary renal tissue ablation system 100 according to embodiments of the present disclosure. System 100 may include an element 102 for providing power to an electrode (not shown) disposed about and/or within an elongate shaft 104 and, optionally, within a sheath or guide catheter 106. A proximal end of element 102 may be connected to a control unit 108, which supplies the necessary electrical energy to activate the one or more electrodes at or near a distal end of the element 102. In some instances, return ground pads 110 may be supplied on the legs or at another conventional location on the patient's body to complete the circuit. In other embodiments, the element 102 may include one or more pairs of bipolar electrodes. The control unit 108 may include monitoring elements to monitor parameters such as power, temperature, voltage, pulse size and/or shape and other suitable parameters as well as suitable controls for performing the desired procedure. In some instances, the control unit 108 may control a radio frequency (RF) electrode. The electrode may be configured to operate at a frequency of approximately 460 kHz. It is contemplated that any desired frequency in the RF range may be used, for example, from 450 - 500 kHz. However, it is contemplated that different types of energy outside the RF spectrum may be used as desired, for example, but not limited to microwave energy.

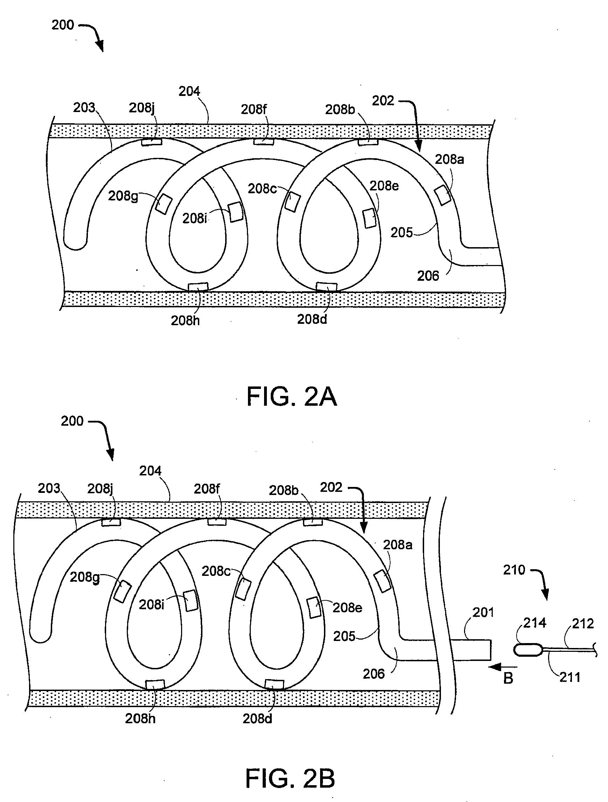

FIGS. 2A and 2B illustrate a portion of a tissue ablation device 200, in accordance with an embodiment of the present disclosure. As shown, the ablation device 200 may be introduced within a body lumen having a vessel wall 204. The vessel wall 204 may be surrounded by local body tissue, which may comprise adventitia and connective tissues, nerves, fat, fluid, etc., in addition to the muscular vessel wall. A portion of the surrounding tissue may be the desired treatment region. In an exemplary embodiment, the ablation device 200 may be introduced within a renal artery, which may provide treatment for a variety of medical conditions such as, but not limited to, hypertension.

The ablation device 200, as shown in FIG. 2A, may include an elongate guide member 202 having a long, thin, flexible tubular configuration. A person skilled in the art will appreciate that other suitable configurations such as, but not limited to, rectangular, oval, irregular, or the like may also be contemplated. In addition, the guide member 202 may include a proximal section 201 (shown in FIG. 2B), a distal section 203, and a lumen 206 extending between them. The distal section 203 may be adapted to rest within a body lumen having vessel wall 204, while the proximal section 201 may remain outside the patient's body. In certain instances, the proximal section 201 of the elongate guide member 202 may include a hub attached thereto for connecting other diagnostic and/or treatment devices for providing a port for facilitating other interventions.

In an embodiment, the distal section 203 may be configured to alter between a released configuration, in which distal section 203 takes on the form of a helix, and a restrained configuration in which distal section 203 is held within a restraining device. In general, distal section 203 is formed as a helix, so that when free of restraint, distal section 203 reverts to a helical shape. When subjected to restraint, however, such as could be provided by withdrawing distal section 203 into a straight restraining tube, that section may flex distal section 203 elastically to assume the shape of the restraining tube. Construction and formation of distal section 203, as well as the operation and achievement of the released and restrained configurations, are all described herein. Distal section 203 extends radially outward from the axis of proximal section 201 to contact the vessel wall 204. To accomplish that purpose, the outside diameter of the helix formed by distal section 203 may be slightly greater than the inside diameter of vessel wall 204. Those skilled in the art will appreciate that the released configuration (i.e., released state) of the distal section 203 may include any suitable configuration having an outer diameter relatively larger than the outer diameter of the restrained configuration (i.e., restrained state).

In an embodiment, the sidewall 205 may include multiple apertures 208a, 208b, 208c, 208d, 208e, 208f, 208g, 208g, 208h, 208i, and 208j (collectively apertures 208). In the illustrated embodiment, apertures 208 may be rectangular, with the short dimension of the rectangle sized about 30% of the diameter of the lumen 206. It will be understood that other shapes, such as circular, elliptical, or the like, as well as alternative dimensions of the apertures 208 may also be contemplated, without departing from the scope and spirit of the present disclosure. In addition, alterations to the number of apertures 208 are also contemplated. For example, the sidewall 205 may include one, two, three, four, five, six, seven, eight, nine, ten, or more apertures 208.

Apertures 208 may be disposed longitudinally and/or circumferentially along the sidewall 205. The desired ablation pattern may dictate the disposition and spacing of apertures, which may be uniform or irregular, or any other design required to achieve the therapeutic result. In particular, the apertures 208 may face radially out from the distal section 203, placing apertures 208 in direct contact with the vessel wall 204 when the guide member is in the released configuration. The illustrated embodiment, for example, would produce a spiral pattern of ablation, with individual ablation sites generally uniformly spaced. In an alternative embodiment, the apertures 208 may be take the form of pores (not shown), which may be distributed along at least 80% of the length of the distal section 203. Pores may be distributed circumferentially and longitudinally along the distal section 203. Pores may be generally circular, sized at about 0.05 inch each. Larger or smaller pores and pores of different and varying shapes are also be contemplated.

Apertures 208 are open to the surrounding environment, and thus fluids, including blood, can flow inside of distal section 203. As discussed further below, fluids can also be introduced through the proximal end of ablation device 200, for cooling or other purposes such as to help with electrical conduction.

The elongate guide member 202 may include a lumen 206 extending between the proximal section 201 and the distal section 203. In an embodiment, the lumen 206 may include a guidewire lumen and/or one or more auxiliary lumens. The lumens may have a variety of configurations and/or arrangements. For example, the guidewire lumen may extend the entire length of the elongate guide member 202 such as in an over-the-wire catheter or may extend only along a distal portion of the elongate guide member 202 such as in a single operator exchange (SOE) catheter. These examples are not intended to be limiting, but rather examples of some optional configurations. In certain instances, the lumen 206 may provide entrance to a variety of components such as, but not limited to, temperature sensor/wire, an infusion lumen, radiopaque marker bands, fixed guidewire tip, and/or other components to facilitate the use and advancement of the device 200 within the vasculature.

As shown in FIG. 2B, the ablation device 200 may further include an electrode catheter 210, a flexible, tubular device which may include an ablation element 214 at the distal end 211 of a shaft 212. The electrode catheter 210 may be configured to be received within lumen 206. Ablation element 214 may include an energy-delivering device such as an electrode, configured to deliver an ablation energy, which can take the form of RF, microwave, or ultrasonic energy. Alternatively, ablation element 214 may be configured to perform cryogenic or alternate forms of ablation.

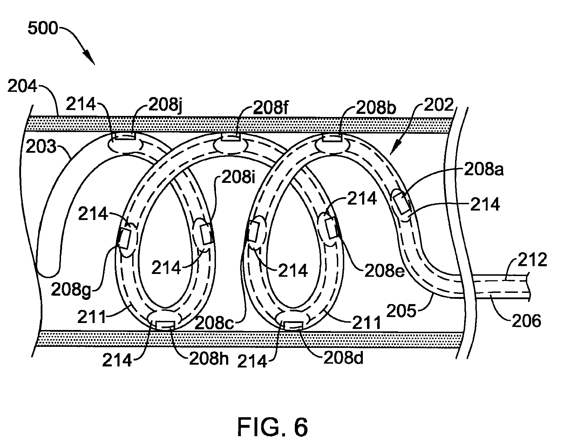

A number of alternatives to the electrode catheter 210 can be employed. For example, two or more electrodes 214 may be provided (e.g., as shown in FIG. 6), spaced on shaft 212 a distance equivalent to the spacing of the apertures 208. In that manner, multiple ablation sites could be treated the same time. Saline solution may be introduced into the distal section 203 to enhance electrical conductivity. A further alternative structure for the electrode catheter 210 could the spacers, designed to ensure a desired distance between the electrodes 214 and the vessel wall 204.

In addition, the shaft 212 may be advanced through the proximal section 201 of the guide member 202, while the distal section 203 is in the released configuration, allowing the ablation element 214 to be placed near the aperture 208. Next, a predetermined amount of energy, RF energy, for example, may be delivered to the ablation element 214. In certain instances, a power element such as conductive wire or the like may join a portion of the ablation element 214 to a power source, which may be disposed proximally outside the patient's body. In certain instances, such power elements may be disposed within a lumen (not shown) of the shaft 212.

It is contemplated that the stiffness of the elongate guide member 202 may be modified to form ablation device 200 for use in various vessel diameters. To this end, the material used for manufacturing the elongate guide member 202 may include any suitable biocompatible material such as, but are not limited to, polymers, metals, alloys, either in combination or alone. The material employed may have enough stiffness for use in various lumen diameters, and sufficient flexibility to maneuver through tortuous and/or stenotic lumens, avoiding any undesirable tissue injuries.

In particular, the material used to manufacture the distal section 203 may include an elastic material, which may allow the distal section 203 to return to a preset released) for deployment following passage in a restrained configuration through a delivery device. One class of such material may include shape memory alloys such as Nitinol and the like. Other suitable materials such as metals, polymers, composites, and the like may also be contemplated, without departing from the spirit and scope of the present disclosure.

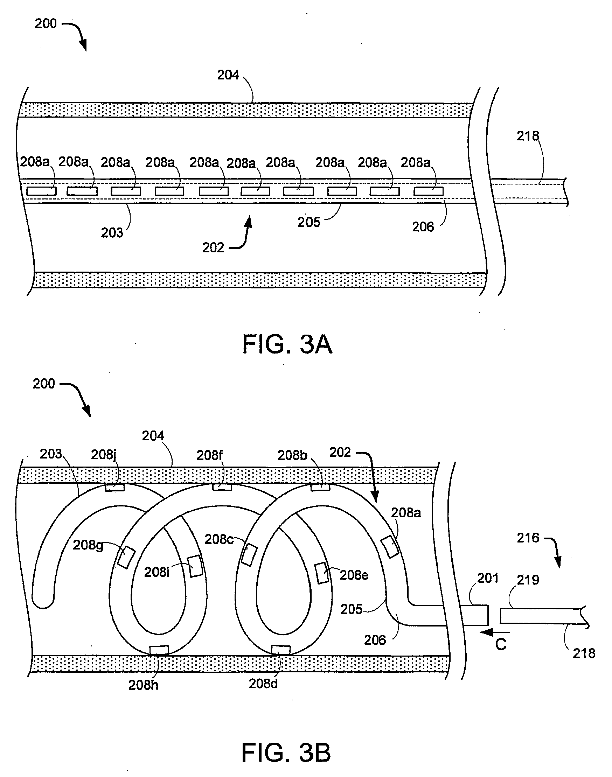

FIG. 3A illustrates distal section 203 in its restrained state. There, a straightening member, which could be a mandrel 218, is inserted into distal section 203. The mandrel has a slightly smaller diameter than distal section 203, and may be considerably stiffer, enabling it to hold distal section 203 in a linear form. That configuration is obtained pushing mandrel into distal section 203, straightening the helical loops of distal section 203. I. Mandrel 218 generally may match the cross-section of distal section 203, and thus in the illustrated embodiment, mandrel 218 exhibits a circular, generally uniform, cross-section. In addition, the diameter of the mandrel 218 may be adapted to allow a generally frictionless movement of the mandrel 218 within the lumen 206. Pulling mandrel 218 proximally from distal section 203 allows that section to resume its helical shape.

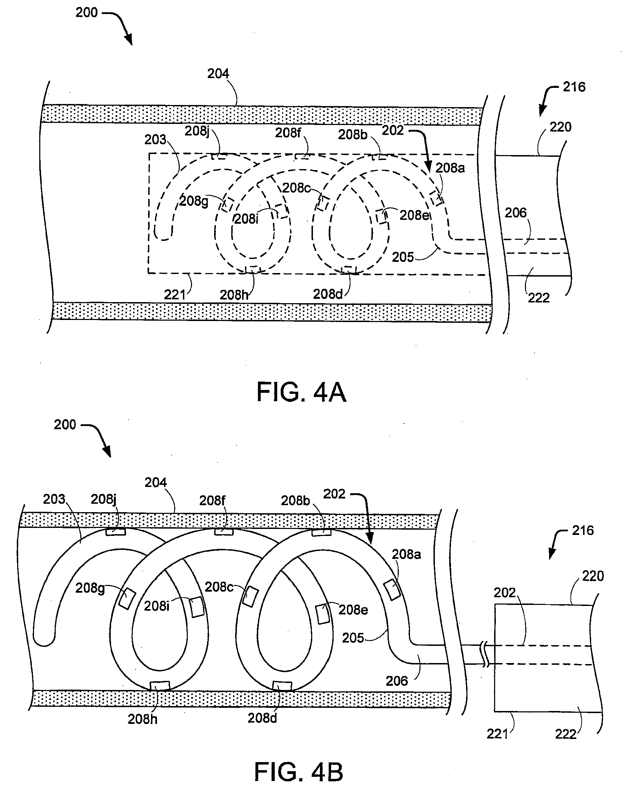

FIG. 3B illustrates the action required to return distal section 203 from a released configuration to a restrained configuration. Advancing mandrel 218 in direction C progressively may force the helical loops of distal section 203 to straighten, finally assuming the restrained configuration of FIG. 3A. FIGS. 4A and 4B illustrate an alternative embodiment. There, rather than employing a device such as mandrel 218 which is inserted into distal section 203 to straighten the helical curves, an external sheath is slid over distal section 203 to achieve that result. Thus, as seen in FIG.4A, this embodiment includes guide catheter 220, having an interior diameter sized to accommodate distal section 203 in a restrained state. It should be noted that the restrained state does not need to be completely straight, as was accomplished in the embodiment of FIG. 3A. Rather, the restrained state may be achieved by a partially straightened distal section 203. Those of skill in the art will understand that sufficient straightening may be achieved if the guide catheter 220 has a diameter sufficiently small to navigate the anticipated target vasculature between the catheter entry point and the application site.

Removal of guide catheter 220, as shown in FIG.4B, may allow the helical distal section 203 to deploy, placing apertures 208 into contact with the vessel wall 204. In the restrained configuration, the walls of the guide catheter 220 may provide a continuous compression force, allowing the device 200 to navigate through the body vessel. Thus, guide catheter 220 must be constructed of material sufficiently rigid to withstand the restoring force exerted by the helical loops of distal section 203 in the restrained configuration.

Material used for manufacturing the guide catheter 220 may include suitable biocompatible materials such as, polymers, metals, or alloys, either in combination or by themselves. It will also be understood by those in the art that in addition to the stiffness requirement mentioned above, the material employed in guide catheter 220 must also exhibit sufficient flexibility to maneuver through tortuous and/or stenotic lumens. Materials that combine those characteristics include Pebax, Arnitel, Hytrel, PBT blends, and polyether-urethane, for example. The catheter may, for example, include a wall embedded with braids or a wall made of slot tubes.

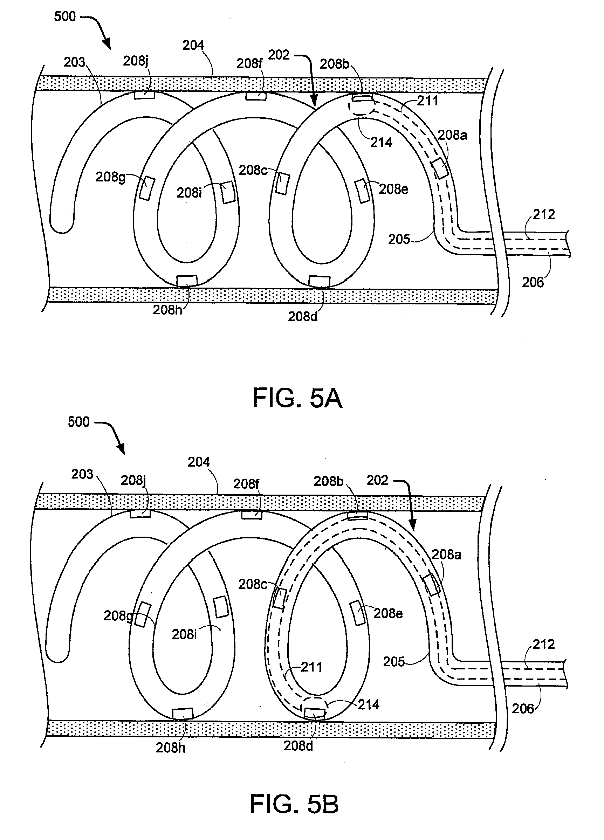

FIGS. 5A and 5B illustrate an exemplary method of using the ablation device 200 to ablate a body tissue such as nerve tissue proximate a renal artery. In one embodiment, the method may include preparing the guide member 202 for delivery inside the patient body. The pre-defined helical distal section 203 of the guide member 202 may be placed in the restrained configuration, by inserting the mandrel 218 into the lumen 206. That operation straightens the helical loops, facilitating navigation of the device 200 to the therapeutic site. Guide member may be introduced within the patient body percutaneously into a blood vessel or using a natural anatomical opening such as the rectum, ureter, anus, or the like. The guide member 202 may then be maneuvered though the body passages to reach a target area such as a renal artery. Once the distal section 203 of the guide member 202 reaches the target site, the straightening member 216 may be retracted, configuring the distal section 203 in the released state. That state returns distal section 203 to the helical configuration shown in FIG. 5A, and the resulting relaxation may bring apertures 208 into contact with the vessel wall 204.

Next, the electrode catheter 210 may be aligned with the lumen 206 of the guide member near the proximal section 201 (shown in FIG. 2B). The distal end 211 of the electrode catheter 210 may be inserted within the lumen 206. A physician may maneuver the electrode catheter 210 within the lumen 206 until the ablation element 214, disposed at the distal end 211 of the electrode catheter 210, reaches a first position, such as aperture 208a, for example (FIG. 5A). Then, the ablation element 214 may be activated by delivering RF energy, for example, which may ablate the tissue adjacent the first position. In some instances, saline may be flushed through the lumen 206, providing electrical conductivity to the ablation element 214 at first position, thus facilitating tissue ablation. Saline may also be introduced to the ablation site for the purposes of cooling.

The electrode catheter 210 may be further advanced within lumen 206 to reach a second position, which may be at aperture 208d, as shown in FIG. 5B. Next, the ablation element 214 may be activated by provide the RF energy through an external power source (not shown). This may ablate the tissue adjacent the second position, i.e., aperture 208d. The ablation element may further travel within the lumen 206 to provide a desired ablation and or lesion pattern.

Those skilled in the art will recognize that the present disclosure may be manifested in a variety of forms other than the specific embodiments described and contemplated herein. Accordingly, departure in forma and detail may be made without departing from the scope and spirit of the present disclosure as described in the appended claims.

## Claims

1. A renal nerve ablation system (100; 200; 500), comprising:
a guide member (202) having a distal section (203) with a plurality of openings (208) formed therein;
wherein the distal section (203) is designed to shift between a first configuration and a second configuration;
wherein the distal section (203) is biased to be in the second configuration;
wherein the distal section (203) includes one or more loops when in the second configuration;
an electrode catheter (210) slidably disposable within the guide member (202), the electrode catheter (210) having an ablation element (214) disposed thereon; and
a mandrel (218) removably disposable within the guide member (202), the mandrel (218) being designed to hold the distal section (203) in the first configuration.

2. The system of claim 1, wherein the second configuration is a helical configuration.

3. The system of claim 2, wherein the distal section (203) in the helical configuration includes at least one loop.

4. The system of claim 2, wherein the distal section (203) in the helical configuration includes at least two loops.

5. The system of any one of claims 1 to 4, wherein the plurality of openings (208) includes a plurality of apertures (208) that face radially outward when the distal section (203) is in the second configuration.

6. The system of claim 5, wherein the plurality of apertures (208) are spaced from each other at a uniform distance.

7. The system of any one of claims 1 to 4, wherein the plurality of openings (208) includes a plurality of pores.

8. The system of claim 7, wherein the pores are distributed along at least 80% of a length of the distal section (203).

9. The system of claim 7 or claim 8, wherein the pores are distributed circumferentially and longitudinally along the distal section (203).

10. The system of any one of claims 1 to 9, wherein the ablation element comprises two or more electrodes (214) spaced on a shaft (212) of the electrode catheter (210) by a distance equivalent to a spacing of the openings (208).

11. The system of claim 10, wherein the electrode catheter (210) comprises spacers designed to ensure a desired distance between the electrodes (214) and a vessel wall (204).
